# NEUE EUROPÄISCHE PATENTSCHRIFT

(11) **EP 0 320 696 B2**
(45) Veröffentlichungstag und Bekanntmachung des Hinweises auf die Entscheidung über den Einspruch: **15.05.1996**
(45) Hinweis auf die Patenterteilung: 07.04.1993
(21) Anmeldenummer: 88119923.6
(22) Anmeldetag: 30.11.1988
(51) Int. Cl.: A61H 33/02, A47K 3/022, B08B 9/06, A61H 33/00

(54) **Wirbeldüsenwanne mit selbsttätiger Systemvorspülung**
Whirlpool bath with automatic pre-rinsing system
Baignoire de bain à remous comprenant un système de prérinçage automatique

(30) Priorität: 15.12.1987 DE 3742432
(43) Veröffentlichungstag der Anmeldung: 21.06.1989
(60) Teilanmeldung: 92103827.9
(73) Patentinhaber: HOESCH Metall + Kunststoffwerk GmbH & Co., D-52304 Düren (DE)
(72) Erfinder: Klotzbach, Manfred, D-5160 Düren (DE)
(74) Vertreter: Langmaack, Jürgen, Dipl.-Ing.

(56) Entgegenhaltungen:
- EP-A- 0 164 068
- EP-A- 0 233 753
- DE-A- 3 447 772
- DE-A- 3 544 002
- DE-A- 3 635 329
- DE-A- 3 715 010
- DE-C- 3 607 788
- GB-A- 2 146 897
- AUSZÜGE AUS DEN GEBRAUCHS- MUSTERN, Heft 24, 12. Juni 1986 WILA VERLAG, München Seite 1007

## Beschreibung

Die Erfindung betrifft eine Wanne mit wenigstens einer im Wandbereich eingebauten Düse zum Einleiten eines Wasser-Luft-Gemisches, die über eine Druckleitung mit der Druckseite einer Pumpe verbunden ist, deren Saugseite über eine Rohrleitung mit einer Ansaugöffnung im Bodenbereich der Wanne in Verbindung steht.

Wannen der vorstehend bezeichneten Art, die in der Regel ein Fassungsvermögen einer normalen Badewanne aufweisen, werden in der Weise betrieben, daß jeweils für ein "Wirbelbad" die Wanne gefüllt wird und nach dem Baden die Wanne wie üblich entleert wird. Hierbei muß dafür Sorge getragen werden, daß beim Entleeren der Wanne auch die Rohrleitungen des Pumpsystems einschließlich der Pumpe ebenfalls entleert werden, um das Entstehen von Keimherden zu verhindern.

Bei Wannen der eingangs bezeichneten Art mit größerem Füllvolumen, bei denen die Wannen- bzw. Beckenfüllungen über einen längeren Zeitraum beibehalten werden und mehrfach benutzt werden, sind im Rohrleitungssystem Einrichtungen zur Konditionierung des Wassers, insbesondere Filter, vorgesehen, um eine Keimbildung im Wasser weitgehend zu unterbinden. Auch bei derartigen Becken ist in gleicher Weise beim Wasserwechsel zunächst für eine vollständige Entleerung auch des Rohrleitungssystems zu sorgen, um bei einer Neufüllung eine Kontaminierung des frisch eingelassenen Wassers zu vermeiden.

Aus der DE-C2-34 20 714 ist eine Wirbeldüsenwanne vorbekannt, bei der das Rohrleitungssystem über ein System von Niveaufühlern und steuerbaren Ventilen so ausgerüstet ist, daß bei einer Befüllung zunächst das gesamte Rohrleitungssystem mit Frischwasser durchgespült wird, so daß etwa noch vorhandene Altwasserreste in die Ablaufleitung ausgespült werden und somit bei Inbetriebnahme des im Umwälzvenfahren arbeitenden Wirbeldüsensystems nicht mehr in die Wannenfüllung gelangen können.

Es hat sich nun gezeigt, daß die Durchspülung mit Frischwasser über einen längeren Zeitraum gesehen nicht immer ausreicht, so daß das gesamte wasserführende Rohrleitungssystem in einem gesonderten Reinigungsgang, bei dem mit dem Frischwasser zugleich eine Reinigungs- und/oder Desinfektionslösung in die Wanne eingefüllt wird, von Zeit zu Zeit behandelt werden muß. Dieser zusätzliche Arbeitsgang wird insbesondere bei Wirbeldüsenwannen, die im privaten Bereich eingesetzt sind, entweder vollständig unterlassen oder in so großen Zeitabständen vorgenommen, daß die Gefahr einer Kontaminierung der Wannenfüllung bei Benutzung über Keimherde im Rohrleitungssystem nicht ausgeschlossen werden kann.

Der Erfindung liegt die Aufgabe zugrunde, eine Wanne der eingangs bezeichneten Art so auszugestalten, daß bei jeder Inbetriebnahme der Wirbeldüsenwanne und zwar sowohl bei einer Neufüllung als auch bei einer Inbetriebnahme des Wirbeldüsensystems bei vollständig gefüllter Wanne eine Durchspülung des gesamten Rohrleitungssystems getrennt vom Wanneninnenraum und unter Zugabe eines Reinigungs- und/oder Desinfektionsmittels möglich ist.

Diese Aufgabe wird gemäß der Erfindung mit den im Anspruch 1 angegebenen Mitteln gelöst. Die Verschließbarkeit der Düsen gegenüber dem Wanneninnenraum macht es möglich, das gesamte Rohrleitungssystem für sich mit Frischwasser oder auch mit einer Reinigungsmittel- und/oder Desinfektionsmittellösung vor jeder Inbetriebnahme des Wirbeldüsensystems zu durchspülen. Dies kann sowohl bei einer Neufüllung der Wanne geschehen als auch bei einer bereits vollständig gefüllten Wanne. Die Überströmleitung zwischen Düse und Saugleitung ermöglicht hier den Spülkreislauf. Wird die Überströmleitung gegenüber der Saugleitung abgesperrt, kann bei geöffneter Düse die Wanne im Wirbelbetrieb benutzt werden. Die Entlüftungsöffnung gestattet es, bei vollständig entleerter Wanne und vollständig entleertem Rohrleitungssystem bei einer Neubefüllung der Wanne und/oder bei einem gesonderten Reinigungsvorgang mit Hilfe der Pumpe das Rohrleitungssystem zunächst vollständig zu befüllen und hierbei zu entlüften. Die Einspeisung für das Reinigungs- und/oder Desinfektionsmittel, nachstehend nur Reinigungsmittel bezeichnet, erfolgt hierbei zweckmässigerweise in Strömungsrichtung gesehen vor der Pumpe, so daß bereits in der Pumpe selbst eine einwandfreie Durchmischung des Reinigungsmittels mit dem Wasser bewirkt wird.

In zweckmäßiger Ausgestaltung der Erfindung ist vorgesehen, daß die Überströmleitung gegenüber der Saugleitung durch ein vorzugsweise steuerbares Absperrventil verschließbar ist. In weiterer zweckmäßiger Ausgestaltung der Erfindung ist vorgesehen, daß die Überströmleitung in Strömungsrichtung der Düse mit einer Zweigleitung versehen ist, die durch ein vorzugsweise steuerbares Absperrventil verschließbar ist und die in das Wannenablaufrohr mündet. Diese Ausgestaltung erlaubt es, für die unterschiedlichen Betriebsweisen eindeutige Druckverhältnisse im Rohrleitungssystem einzustellen. Durch die Anordnung der absperrbaren Zweigleitung ist es möglich, auch den unterhalb des Wannenniveaus liegenden Teil der Überströmleitung vollständig zu entleeren. Wird im Reinigungsbetrieb das Absperrventil der Überströmleitung vollständig geöffnet und das Absperrventil der Zweigleitung nicht ganz geschlossen, so ist es möglich, bei gleichzeitiger Frischwasserzufuhr über die Ansaugöffnung im Bodenbereich der Wanne aus dem Wanneninnenraum immer einen Teil aus dem im Kreislauf geführten Wasser zu entfernen und dann nach Abschluß des Reinigungsvorganges durch Absperren der Überströmleitung gegenüber der Saugleitung und gleichzeitigem vollständigen Öffnen des Absperrventils in der Zweigleitung, das Rohrleitungssystem mit Frischwasser zu durchspülen und hierbei die Reinigungsmittellösung vollständig auszuspülen. Für den Wirbelbetrieb wird dann auch noch das Absperrventil der Zweigleitung geschlossen, so daß nach vollständiger Füllung des Wanneninnenraumes bei nunmehr geöffneter Düse der Wirbelbetrieb durchgeführt werden kann.

In zweckmäßiger Ausgestaltung ist die Entlüftungsöffnung mit einem Absperrventil, vorzugsweise einem Schwimmerventil absperrbar ausgebildet, so daß nach dem vollständigen Entlüften der Überströmleitung das Rohrleitungssystem im Kreislauf mit Wasser und/oder einer Reinigungsmittellösung beaufschlagt werden kann.

In weiterer vorteilhafter Ausgestaltung der Erfindung ist ferner vorgesehen, daß die Düse einen Sperrkörper aufweist, der die Druckleitung gegenüber dem Wanneninnenraum bei einem Pumpendruck zwischen Null und einem ersten geringen Druckniveau verschlossen hält und bei Überschreiten des ersten Druckniveaus selbsttätig öffnet. Während der vorstehend geschilderte Reinigungsbetrieb auch mittels von Hand verschließbarer Düsen möglich ist, erlaubt diese Ausgestaltung einen automatisierten Betrieb, insbesondere dann, wenn über eine entsprechende Steuerschaltung auch die Absperrventile in der Überströmleitung und in der Zweigleitung ansteuerbar ausgebildet sind und in Abhängigkeit vom Druck in der Druckleitung, oder in Abhängigkeit von einem Füllstandsniveau im Innenraum oder aber auch über ein Zeitprogramm betätigt werden. Besonders zweckmäßig ist es hierbei, wenn in weiterer Ausgestaltung der Erfindung eine in ihrer Drehzahl veränderbare, vorzugsweise stufenlos regelbare Pumpe vorgesehen ist, die in Verbindung mit einem Niveaufühler beim Überschreiten eines Mindestfüllniveaus in der Wanne den Antriebsmotor der Pumpe auf eine Förderleistung einschaltet, die einem Pumpendruck unterhalb des ersten Druckniveaus entspricht. Hierdurch ist gewährleistet, daß bei druckabhängig selbsttätig öffnender Düse und entsprechend geöffneten Absperrventilen in der Überströmleitung und/oder der Zweigleitung die im Rohrleitungssystem über die Pumpe geführte Flüssigkeit nicht in den Wanneninnenraum eintreten kann.

Während es grundsätzlich möglich ist, das Rohrleitungssystem über eine gesonderte Ansaugöffnung mit dem Wanneninnenraum zu verbinden, ist in einer bevorzugten Ausgestaltung der Erfindung vorgesehen, daß die mit dem Wanneninnenraum in Verbindung stehende Ansaugöffnung der Saugleitung durch die wannenseitige Öffnung einer kammerartigen Wannenablaufarmatur gebildet wird, in der der Wannenverschluß unterhalb der Einmündung der Saugleitung angeordnet ist, daß die Überströmleitung oberhalb des Wannenverschlusses und die Zweigleitung unterhalb des Wannenverschlusses in die Wannenverschlußarmatur einmündet. Hierdurch ist es möglich, die ohnehin notwendige Ablauföffnung mit der Ansaugöffnung der Saugleitung der Pumpe zu kombinieren und zugleich auch die Überströmleitung bzw. deren Zweigleitung mit der Saugleitung bzw. dem Abflußrohr in einer Armatur zusammenzufassen. Durch die Anordnung der Einmündungen der Saugleitung und der Überströmleitung in die kammerartige Wannenablaufarmatur oberhalb des Wannenverschlusses ist es bei entsprechender Ausgestaltung der Strömungsquerschnitte ferner möglich, ohne einen zusätzlichen Verschluß der Ansaugöffnung das Rohrleitungssystem auch mit einer Reinigungsmittellösung durchzuspülen, ohne daß dieses Reinigungsmittel in den Wanneninnenraum eintreten kann. Dies ist insbesondere dann gewährleistet, wenn bei geringfügig geöffnetem Absperrventil der über die Saugleitung im Bereich der kammerartigen Anschlußarmatur anliegende Unterdruck so bemessen ist, daß zusätzlich zu den Flüssigkeitsmengen aus der Überströmleitung auch eine geringe Teilmenge über die Ansaugöffnung aus dem Wanneninnenraum abgezogen wird.

In weiterer Ausgestaltung der Erfindung ist die Wannenablaufarmatur, die an die Ablauföffnung der Wanne anschließbar ist, so ausgebildet, daß sie eine unterhalb des Wannenbodens angeordnete Strömungskammer aufweist, die mit einer oberen Öffnung versehen ist, die als Ablauföffnung in den Wanneninnenraum mündet und die mit einer unteren Öffnung versehen ist, die in das Ablaufrohr mündet und die mit einem von außen betätigbaren Ventilkörper verschließbar ist, daß ferner die Strömungskammer oberhalb des Ventilkörpers zwei vorzugsweise diametral gegenüberliegend angeordnete Strömungsöffnungen aufweist, von denen eine an eine Zulaufleitung und die andere an eine Saugleitung einer Pumpe anschließbar ist. Eine derart ausgebildete Wannenablaufarmatur hat den Vorteil, daß die Ablauföffnung zugleich als Ansaugöffnung für die Saugleitung der Pumpe dient, so daß bei geschlossener Zulaufleitung Wasser aus dem Wanneninnenraum über die Pumpe durch die Düsen im Kreislauf geführt werden kann (Wirbelbetrieb). Wird dagegen die Zulaufleitung geöffnet, so wird je nach Abstimmung der Querschnitte zwischen der Saugleitung einerseits und der Zulaufleitung andererseits, wobei die Zulaufleitung zweckmäßigerweise einen geringeren Querschnitt aufweist, die aus der Zulaufleitung zuströmende Flüssigkeitsmenge strahlförmig unmittelbar in die Saugleitung eingeleitet, so daß ein Überströmen der Flüssigkeit aus der Zulaufleitung in den Wanneninnenraum vermieden wird. Vielmehr wird eine geringe Schleppströmung erzeugt, die eine Teilmenge aus der Wanne selbst über die Saugleitung mit abzieht. Bei Spülbetrieb, insbesondere bei einem Spülbetrieb mit Reinigungsmittellösung wird somit vermieden, daß ein Teil der Reinigungsmittellösung in den Wanneninnenraum gelangen kann.

In einer Ausgestaltung der Erfindung ist vorgesehen, daß die an die Zuleitung anschließbare Öffnung einen in die Strömungskammer gegen die andere Öffnung hineinragenden Rohransatz aufweist. Hierdurch arbeitet die aus der Zulaufleitung kommende Flüssigkeitsmenge nach Art einer Strahlpumpe gegenüber der Ablauföffnung der Wanne.

In einer anderen Ausgestaltung der Erfindung ist vorgesehen, daß die mit dem Wanneninnenraum verbundene Ausaugöffnung über einen Rohransatz mit darunterliegender Ablauföffnung verbunden ist, der einen geringeren Durchmesser als der Durchmesser der Strömungskammer aufweist und daß die Wandung des Rohreinsatzes auf Seiten der mit der Saugleitung verbindbaren Öffnung der Strömungskammer mit wenigstens einer Durchtrittsöffnung versehen ist. Bei dieser Anordnung ist die als Ablauföffnung der Wanne dienende Öffnung der Strömungskammer gegenüber der Zulaufleitung abgeschirmt, so daß ein direkter Übertritt des aus der Zulaufleitung zuströmenden Wassers in den Wanneninnenraum vermieden ist. Gleichwohl erlaubt der Rohreinsatz mit seinen der Öffnung der Saugleitung zugekehrten Durchtrittsöffnungen einen ungehinderten Durchtritt des Wassers aus dem Wanneninnenraum in die Saugleitung, so daß der normale Wirbelbetrieb sichergestellt ist.

Die Erfindung wird anhand schematischer Zeichnungen an Ausführungsbeispielen näher erläutert.

Es zeigen:
- Fig. 1: eine Whirlpool-Wanne mit Rohrleitungssystem,
- Fig. 2: einen vertikalen Schnitt durch eine Düse,
- Fig. 3: einen Schnitt durch die Vorkammer der Düse gem. Fig. 2 entlang der Linie III-III in Fig. 2,
- Fig. 4: einen Vertikalschnitt durch eine Ablaufarmatur.

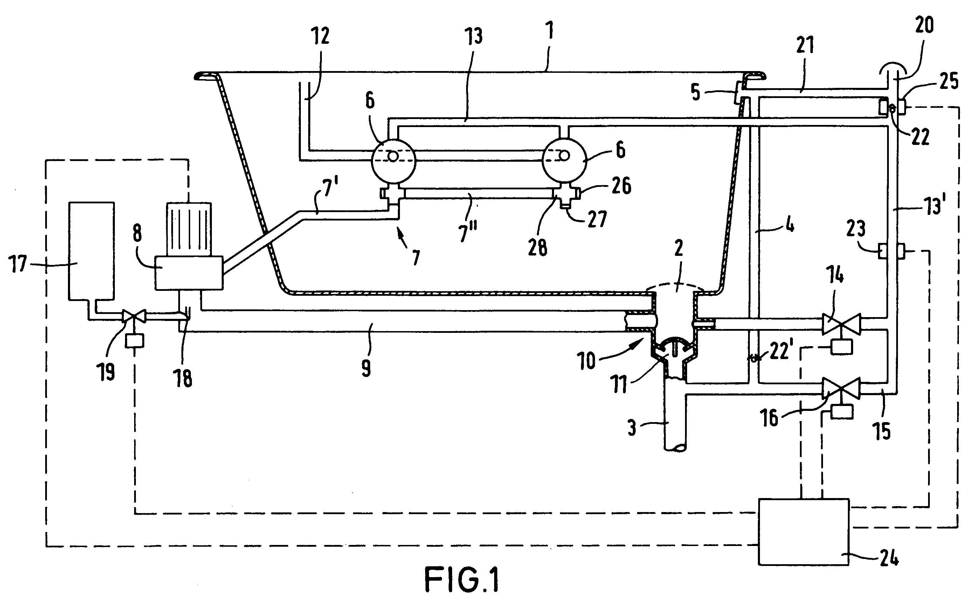

Die in Fig. 1 dargestellte Wirbeldüsenwanne 1 in Form einer etwas größeren Badewanne ist in üblicher Weise mit einer Ablauföffnung 2 im Bodenbereich versehen, die mit einem Ablaufrohr 3 in Verbindung steht. Mit dem Ablaufrohr 3 ist ferner über eine Rohrleitung 4 in üblicher Weise der Wannenüberlauf 5 verbunden.

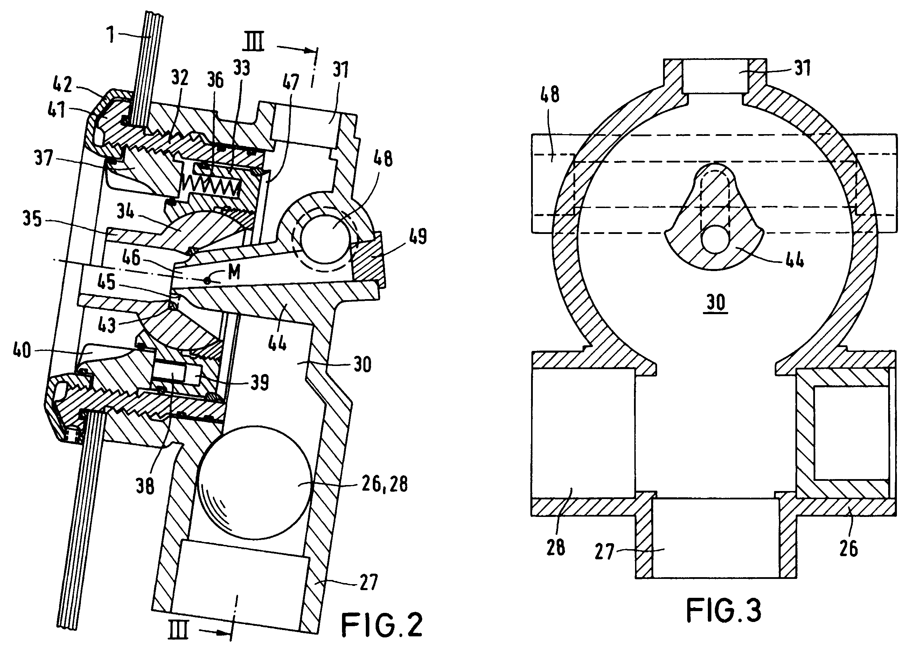

Die Wanne 1 weist nun auf ihren beiden gegenüberliegenden Längsseiten je zwei Düsen 6 auf, durch die ein Wasser-Luft-Gemisch bei gefüllter Badewanne in den Wanneninnenraum strahlförmig eingeleitet werden kann. Die Düsen 6, die anhand von Fig. 2 noch näher erläutert werden, weisen schwenkbare Düsenkörper auf, so daß die Strahlrichtung innerhalb vorgegebener Grenzen frei einstellbar ist.

Die Düsen 6 sind über eine Druckleitung 7 mit der Druckseite einer Pumpe 8 verbunden, die ihrerseits über eine Saugleitung 9 über eine Ablaufarmatur 10 mit der Ablauföffnung 2 in Verbindung steht, die zugleich als Ansaugöffnung für den Pumpenkreislauf dient. Die Ablaufarmatur ist unterhalb der Einmündung der Saugleitung 9 mit einem von außen betätigbaren Ventilkörper 11 gegenüber dem Ablaufrohr 3 verschließbar. Die Pumpe 8 ist hierbei liegend angeordnet, d.h. mit nach unten weisender Ansaugöffnung, so daß bei Stillstand der Pumpe und geöffnetem Ventilkörper 11 die Druckleitung 7 die Pumpe 8 und die Saugleitung 9 vollständig leerlaufen können.

Die Düsen 6 stehen ferner mit einer Luftzuleitung 12 in Verbindung, durch die entweder über die Sogwirkung des in den Wannenraum strömenden Wassers oder aber über einen angeschlossenen Verdichter Luft zusammen mit dem Wasser in Form eines strahlartigen Wasser-Luft-Gemisches in die Wanne eingeleitet werden kann. Der Luftverdichter bzw. die Luftansaugöffnung bei Saugbetrieb, hier nicht näher dargestellt, sind einstellbar, so daß die eingeleitete Luftmenge regulierbar ist.

Die Druckleitung 7 mündet in eine auf der Wannenaußenseite angeordnete Vorkammer der Düsen 6 ein, und zwar von unten. Die Vorkammer jeder Düse 6 ist auf ihrer Oberseite an eine Überströmleitung 13 angeschlossen. Dies wird anhand der Fig. 2 und 3 noch näher erläutert werden.

Die Überströmleitung 13 mündet nun mit ihrem anderen Ende in die Ablaufarmatur 10 oberhalb des Ventilkörpers 11 ein und ist gegenüber dieser über ein Absperrventil 14 absperrbar.

Von der Überströmleitung 13 zweigt eine Zweigleitung 15 ab, die unterhalb des Ventilkörpers 11 in die Ablaufarmatur 10 bzw. das Ablaufrohr 3 einmündet. Auch die Zweigleitung 15 ist über ein Absperrventil 16 gegenüber der Ablaufarmatur bzw. dem Ablaufrohr absperrbar.

Der Wanne ist ferner ein Vorratsbehälter 17 für ein Reinigungs- und/oder Desinfektionsmittel zugeordnet, der über eine Zuleitung 18 vorzugsweise in die Saugleitung 9 einmündet und der über ein Ventil 19 absperrbar ist.

Die Überströmleitung 13 ist ferner mit einem vorzugsweise bis in die Höhe des Wannenrandes geführten Entlüftungsstutzen 20 versehen, der eine Entlüftungsöffnung für die Überströmleitung 13 bildet. Vom Entlüftungsstutzen 20 zweigt eine Querleitung 21 ab, die in die Überlaufleitung 4 einmündet. Durch ein selbsttätiges Ventil, beispielsweise ein Schwimmerventil 22, unterhalb der Querleitung 21 ist sichergestellt, daß nur Leckmengen aus der Überströmleitung 13 in die Überlaufleitung 4 übertreten können.

Der vertikale Teil 13' der Überströmleitung 13 ist mit einem Niveaufühler 23 verbunden, dessen Steuersignal auf eine Steuereinheit 24 aufgeschaltet ist. Dieser Niveaufühler befindet sich gegenüber dem Wannenboden in Höhe einer vorgegebenen Mindestfüllhöhe von beispielsweise 10 cm. Im Entlüftungsstutzen 20 ist unterhalb des Ventils 22 ein weiterer Niveaufühler 25 vorgesehen, der ebenfalls auf die Steuereinheit 24 aufgeschaltet ist.

Die Niveaufühler 23 und 25 sind über die Steuereinheit 24 mit den Antriebsmotor der Pumpe 8 so verschaltet, daß die Pumpe 8 erst eingeschaltet werden kann, wenn die durch den Niveaufühler 23 vorgegebene Mindestfüllhöhe erreicht wird und das auch nur mit einer geringen Förderleistung. Erst bei Erreichen der durch den Niveaufühler 25 vorgegebenen Betriebsfüllhöhe läßt sich die Pumpe mit voller Förderleistung einschalten.

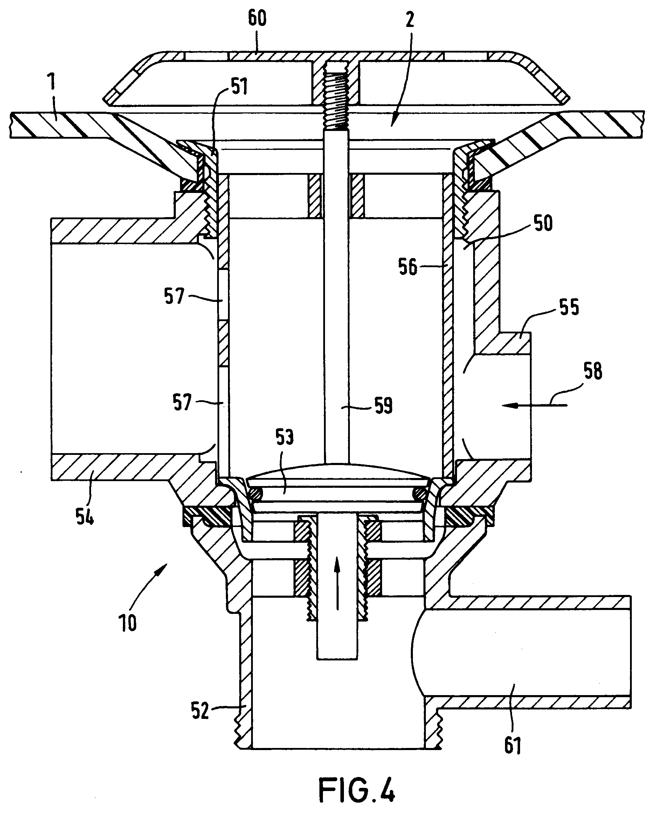

Die Düsen 6 sind verschließbar ausgebildet, was nachstehend anhand von Fig. 2 noch näher erläutert werden wird, so daß bei verschlossenen Düsen 6 und geöffnetem Absperrventil 14 und geschlossenem Absperrventil 16 in der Zweigleitung 15 bei Erreichen der durch den Niveaufühler 23 vorgegebenen Mindestfüllhöhe die Pumpe 8 Flüssigkeit im Kreislauf über die Druckleitung 7, die Überströmleitung 13, 13' und die Saugleitung 9 pumpen kann. Die Ablaufarmatur 10 ist hierbei so ausgebildet, daß keine oder nur geringe Flüssigkeitsmengen aus dem Wanneninnern in die Saugleitung 9 gelangen, was nachstehend anhand von Fig. 4 noch näher erläutert werden wird. Beim Öffnen des Absperrventils 19 kann nun ein Reinigungs- und/oder Desinfektionsmittel zudosiert werden, so daß eine Reinigungsmittellösung im Kreislauf durch das Rohrleitungssystem geführt werden kann, ohne daß der Wanneninnenraum hiermit in Berührung kommt.

Versieht man die Absperrventile 14 und 16 und ggf. das Absperrventil 19 mit steuerbaren Stellantrieben, so lassen sich diese auf die Steuereinheit 24 aufschalten, so daß sowohl die Ansteuerung der Pumpe als auch die Ansteuerung dieser Ventile über die Steuereinheit 24 nach einem vorgegebenen Schaltprogramm erfolgen kann. Hierbei ist es auch möglich, daß während des Reinigungsbetriebes das Absperrventil 16 geringfügig geöffnet wird, so daß immer ein Teilstrom der Reinigungsmittellösung in das Ablaufrohr 3 abgegeben wird und entsprechende Frischwassermengen aus dem Wanneninnenraum in die Saugleitung 9 gelangen. Nach Abschluß der Reinigungsprozedur wird das Absperrventil 16 vollständig geöffnet und das Absperrventil 14 vollständig geschlossen, so daß unter gleichzeitiger Ansaugung von Frischwasser aus dem Wanneninnenraum das wasserführende Rohrleitungssystem, bestehend aus der Saugleitung 9, der Druckleitung 7 und der Überströmleitung 13, 13', mit Frischwasser vollständig durchgespült werden kann. Nach Abschluß dieses Spülvorganges wird das Absperrventil 16 geschlossen.

Da mit Beginn des Reinigungsvorganges die Förderpumpe nur mit geringer Förderleistung arbeitet, ist es zudem möglich, das Rohrleitungssystem druckseitig langsam zu füllen, so daß auch die in der Druckleitung 7 sowie in den Vorkammern der Düsen 6 und der Überströmleitung 13 enthaltenen Luftmengen über den Entlüftungsstutzen 20 entweichen können, bis auch das Überströmsystem vollständig gefüllt ist, hierbei ein Rückflußverhinderer 22' in der Rohrleitung 4 und schließlich das Schwimmerventil 22 schließt.

Während des Reinigungsvorganges kann das Befüllen der Wanne weitergeführt werden, da erst bei Erreichen der durch den Niveaufühler 25 vorgegebenen Betriebsfüllhöhe die Pumpe 8 auf volle Leistung schaltbar ist. Über eine entsprechende Verriegelungschaltung kann sichergestellt werden, daß erst mit Schließen des Absperrventils 16 nach Abschluß des Spülens mit Frischwasser, der Pumpenbetrieb mit voller Leistung möglich ist. Bei von Hand verschlossenen Düsen 6 müssen diese zunächst geöffnet werden. Bei selbsttätig schließenden Düsen reicht der gegenüber dem Reinigungsbetrieb sehr viel höhere Pumpendruck des Wirbelbetriebes aus, die Düsen zu öffnen.

Die Düsen 6 sind im Zulaufbereich mit drei unter einem rechten Winkel zueinander stehenden Anschlußstutzen 26, 27 und 28 versehen, so daß bei der dargestellten Anordnung der zwischen den beiden Düsen verlaufende Teil 7'' der Druckleitung mit den horizontal ausgerichteten Anschlußstutzen 26 und 28 verbunden ist, während der zwischen der Pumpe 8 und der in Strömungsrichtung gesehen ersten Düse liegende Teil 7' der Druckleitung in den nach unten weisenden Anschlußstutzen 27 einmündet. Hierdurch ist ein einwandfreier Ablauf des Wassers beim Entleeren des Rohrleitungssystems gewährleistet.

In Fig. 2 ist nun eine besondere Ausführungsform für eine selbsttätig schließende Düse 6 dargestellt. Die Düse besteht im wesentlichen aus einer Vorkammer 30 mit ihren untenliegenden, horizontal laufenden Anschlußstutzen 26, 28 (vgl. Fig.3) und dem vertikal nach unten weisenden Anschlußstutzen 27. Auf der Oberseite befindet sich ein Anschlußstutzen 31 für den Anschluß der Überströmleitung 13. Die Vorkammer 30 ist gegen die Außenseite der unter einer Neigung von beispielsweise 10° gegenüber der vertikal verlaufenden Seitenwand der Wanne 1 mittels eines an der Innenseite der Wanne eingeschraubten Tragringes 32 unter Abdichtung befestigt. Im Tragring 32,dessen der Vorkammer 30 zugekehrtes Ende als Zylinder ausgebildet ist, ist nun ein als Kolben ausgebildeter und mit Dichtungen versehener Haltering 33 axial verschiebbar geführt.

Im Haltering 33 ist ein als Kugelkörper ausgebildeter Düsenkörper 34 schwenkbar gehalten, dessen rohrförmige, die Strahlrichtung bestimmende Mündung 35 in den Wanneninnenraum weist.

Auf der der Vorkammer 30 abgekehrten Seite ist der Haltering 33 über mehrere gleichmäßig über den Umfang verteilte Druckfederelemente 36 abgestützt, wobei ein Stellring 37 einen Endanschlag definiert. Der stellring 37 ist ferner mit wenigstens einem Führungsstift 38 versehen, der in eine axiale Bohrung 39 im Haltering 33 eingreift, so daß der Haltering 33 in axialer Richtung relativ gegenüber dem Stellring 37 verschiebbar ist. Der Stellring 37 ist hierbei im Tragring 32 über ein Gewinde gehalten, so daß Stellring und Haltering gemeinsam gegenüber dem Tragring verdreht und axial verschoben werden können. Aufgrund der federnden Ankoppelung zwischen Haltering 33 und Stellring 37 kann hierdurch sowohl das axiale Spiel zwischen verschiebbarem Haltering 33 und Stellring 37 als auch innerhalb gewisser Grenzen die Federvorspannung eingestellt werden. Der Stellring 37 ist hierbei mit einem von der Wanneninnenseite her zugänglichen Griffelement 40 versehen, beispielsweise in Form einer Ausnehmung oder eines Steges. Eine den Klemmbund 41 des Tragringes 32 überdeckende Zierkappe 42 dient zugleich als Begrenzung für die axiale Verstellung des Stellringes 37 in Richtung auf die Wanneninnenseite.

Der im Haltering 33 gelagerte Kugelkörper 34 ist auf der der Vorkammer 30 zugekehrten Seite seiner Bohrung mit einem ringförmigen Dichtelement 43 versehen. Durch die Vorkammer 30 ist im wesentlichen in axialer Richtung, bei dem dargestellten Ausführungsbeispiel geringfügig von oben nach unten geneigt, eine Luftzuleitung 44 geführt, die bis in den Bereich des Düsenkörpers 34 reicht. Die Außenfläche 45 der bis in den Bereich des Düsenkörpers 34 ragenden Mündung 46 der Luftzuleitung 44 ist als Kugelfläche ausgebildet und wirkt mit dem ringförmigen Dichtelement 43 zusammen. Bei drucklosem Zustand der Vorkammer 30 wird der Düsenkörper 34 über die Druckfederelemente 36 mit seinem Dichtelement 43 gegen die Außenfläche 45 gepreßt. Der Mittelpunkt des Kugelteils des Düsenkörpers 34 und der als Kugelfläche geformten Außenfläche 45 der Mündung 46 fallen hierbei zusammen, so daß der Düsenkörper 34 in seinem Haltering 33 verschwenkt werden kann, ohne daß das Dichtelement 43 von der Fläche 45 der Mündung 46 abhebt. In jeder beliebigen Winkelstellung des Düsenkörpers 34 bleibt somit die Abdichtung zwischen dem Wanneninnenraum und der Vorkammer 30 erhalten.

Entsprechend der der Vorkammer 30 zugekehrten Flächenteile des Halteringes 33 und des Düsenkörpers 34 bewirkt nun jede Druckbeaufschlagung der Vorkammer 30 eine gegen die Kraft der Druckfederelemente gerichtete Kolbenkraft. Die Kolbenkraft kann jedoch die Schließkraft der Druckfederelemente 36 erst ab einer bestimmten, vorgegebenen Druckhöhe überwinden und den Düsenkörper 34 mit seiner Dichtung 43 von der Gegenfläche 48 der Mündung 46 abheben, so daß aus der Vorkammer 30 das Wasser in den Wanneninnenraum strömen kann. Für eine Wanne mit vorspülbarem Leitungssystem ist die Schließkraft der Druckfederelemente 36 so eingestellt, daß bei einer geringen Mindestförderleistung der zugehörigen Pumpe die Düsen sicher verschlossen sind und erst nach Einschalten der sehr viel höheren Förderleistung für den Wirbelbetrieb die Düsen sicher geöffnet sind.

Durch Verdrehen des Tellringes 37 in Richtung auf die Vorkammer 30 ist es jedoch möglich, die druckabhängige Axialbewegung des Halteringes 33 zu unterbinden und den Düsenkörper 34 gegen die Mündung 46 anzupressen, so daß das Rohrleitungssystem auch mit der vollen Förderleistung der Pumpe durchspült werden kann.

Der als Kolben ausgebildete Haltering 33 ist an seinem der Vorkammer 30 zugekehrten Kantenbereich mit einem Abstreifer 47 versehen, der bei druckloser Vorkammer 30 in diese hineinragt, so daß Kalkablagerungen von der durch den Tragring 32 gebildeten Zylinderfläche abgeschoben werden bzw. nicht entstehen können.

Wenn der Düsenkörper 34 dichtend an der Mündung 46 anliegt, ist zwar die Verbindung zwischen dem Wanneninnenraum und der Vorkammer 30 unterbrochen. Gleichwohl kann bei gefüllter Wanne Wasser in die Luftzuleitung 44 einlaufen. Durch die geneigte Anordnung der Luftzuleitung 44 wird diese jedoch beim Entleeren der Wanne zuverlässig entleert. Bei dem dargestellten Ausführungsbeispiel erfolgt die Verbindung mit der Luftleitung 12 durch seitliche Anschlußstutzen 48, so daß zusätzlich noch die Möglichkeit eines axialen Anschlusses verbleibt, beispielsweise für einen zusätzlichen Luftverdichter. Bei dem dargestellten Ausführungsbeispiel ist die axiale Zuleitung jedoch durch einen Stopfen 49 geschlossen.

Wie in Fig. 3 dargestellt, werden nicht benötigte Anschlußstutzen durch einen Stopfen verschlossen.

Wie Fig. 2 zeigt, sind die beweglichen Teile der Düse jederzeit vom Wanneninnenraum her zugänglich, ohne daß die Anordnung von der Wanne gelöst werden muß. Hierzu ist es lediglich erforderlich, die Zierkappe 42 zu lösen und dann den Stellring 37 zusammen mit dem als Kolben ausgebildeten Haltering 33 herauszuziehen. Die Dichtungen der gegeneinander bewegbaren Teile lassen sich dann erneuern oder ein neues Kolbenteil einsetzen.

In Fig. 4 ist ein Ausführungsbeispiel für die Ablaufarmatur 10 dargestellt. Diese weist eine Strömungskammer 50 auf, die über einen Klemmring 51 mit der Ablauföffnung 2 im Boden der Wanne 1 fest verbunden ist. Die Strömungskammer 50 weist an ihrem unteren Ende einen Anschlußstutzen 52 auf, mit dem sie an die Ablaufleitung 3 (hier nicht dargestellt) angeschlossen ist. Im unteren Bereich der Strömungskammer 50 ist ein Ventilkörper 53 angeordnet, der aus der dargestellten Schließstellung über einen hier nicht näher dargestellten Öffnungsmechanismus in die Öffnungsstellung angehoben werden kann.

Die Strömungskammer 50 ist oberhalb der Ebene des Ventilkörpers 53 mit einer Strömungsöffnung 54 mit großem Durchmesser in Form eines Anschlußstutzens versehen, der eine zweite Strömungsöffnung 55, ebenfalls in Form eines Anschlußstutzens, diametral gegenüberliegend angeordnet ist. Die Strömungsöffnung 55 hat einen geringeren Strömungsquerschnitt als die Strömungsöffnung 54. An die Strömungsöffnung 54 wird die Saugleitung 9 der Pumpe 8 und an die Strömungsöffnung 55 wird die Überströmleitung 13 des anhand von Fig. 1 beschriebenen Rohrleitungssystems angeschlossen.

In der Strömungskammer 50 ist ein Rohreinsatz 56 angeordnet, der einen geringeren Durchmesser aufweist, als die Strömungskammer selbst und der auf seiner der mit der Saugleitung 9 verbindbaren Strömungsöffnung 54 zugekehrten Seite mit einer Vielzahl von Öffnungen 57 versehen ist, während er auf seiner der Strömungsöffnung 55 zugekehrten Seite eine vollständig geschlossene Wandung aufweist. Bei entsprechender Bemessung der Querschnitte wird hierdurch vermieden, daß das beim Spülvorgang in Richtung des Pfeiles 58 über die Strömungsöffnung 55 zuströmende Wasser in den Wanneninnenraum eintreten kann.

Der Ventilkörper 53 weist auf seiner Oberseite eine Verlängerung 59 auf, mit der eine mit Löchern versehene Abdeckkappe 60 verbunden ist. Bei geschlossenem Ventilkörper dient diese Abdeckkappe 60 als Sicherung der Zulauföffnung zur Saugleitung 9. Beim Öffnen des Ventilkörpers wird die Abdeckkappe 60 mit angehoben, so daß ein ausreichender Querschnitt für den Entleerungsvorgang vorhanden ist. Die Löcher 57 im Rohreinsatz 56 sind hierbei so angeordnet, daß auch die mit den Öffnungen 55 und 54 verbundenen Zulaufleitungen vollständig über die Ablaufarmatur 10 entleerbar sind.

Unterhalb des Ventilkörpers 53 ist eine weitere Strömungsöffnung 61, ebenfalls in Form eines Anschlußstutzens vorgesehen, in die bei einer Anordnung gemäß Fig. 1 die Zweigleitung 15 einmündet.

## Patentansprüche

1. Wanne mit wenigstens einer im Wandbereich eingebauten Düse (6) zum Einleiten eines Wasser-Luft-Gemisches, die mit einer Luftzuleitung (12) in Verbindung steht und die über eine Druckleitung (7) mit der Druckseite einer Pumpe (8) verbunden ist, deren Saugseite über eine Rohrleitung (9) mit einer Ansaugöffnung im Bodenbereich der Wanne in Verbindung steht und bei der in Strömungsrichtung gesehen, vor der Düse (6) eine Einspeisung (18) für ein Reinigungsmittel vorgesehen ist, dadurch gekennzeichnet, daß der über die Druckleitung (7) mit der Pumpe (8) in Verbindung stehende, gegenüber der Luftzuleitung (12) abgetrennte Teil der Düse (6) gegen den Wanneninnenraum verschließbar ausgebildet ist und an eine Überströmleitung (13) angeschlossen ist, die mit einer verschließbaren Entlüftungsöffnung (22) versehen ist und die über eine, die Ansaugöffnung (2) enthaltende Wannenablaufarmatur (10) mit der Saugleitung (9) der Pumpe (8) absperrbar in Verbindung steht.

2. Wanne nach Anspruch 1, dadurch gekennzeichnet, daß die Überströmleitung (13) gegenüber der Saugleitung (9) durch ein vorzugsweise steuerbares Absperrventil (14) verschließbar ist.

3. Wanne nach Anspruch 1 oder 2, dadurch gekennzeichnet, daß die Überströmleitung in Strömungsrichtung hinter der Düse (6) mit einer Zweigleitung (15) versehen ist, die durch ein vorzugsweise steuerbares Absperrventil (16) verschließbar ist und die in das Wannenablaufrohr (3) mündet.

4. Wanne nach einem der Ansprüche 1 bis 3, dadurch gekennzeichnet, daß die Entlüftungsöffnung (20) über ein Absperrventil (22), vorzugsweise ein Schwimmerventil, absperrbar ausgebildet ist.

5. Wanne nach einem der Ansprüche 1 bis 4, dadurch gekennzeichnet, daß die Düse (6) einen Sperrkörper aufweist, der die Druckleitung (7) gegenüber dem Wanneninnenraum bei einem Pumpendruck zwischen Null und einem ersten geringen Druckniveau verschlossen hält und bei Überschreiten des ersten Druckniveaus selbsttätig öffnet.

6. Wanne nach einem der Ansprüche 1 bis 5, dadurch gekennzeichnet, daß eine in ihrer Drehzahl veränderbare, vorzugsweise stufenlos regelbare Pumpe (8) vorgesehen ist, daß ein Niveaufühler (23) vorgesehen ist, der beim Überschreiten einer Mindestfüllhöhe in der Wanne (1) den Antriebsmotor der Pumpe (8) auf eine Förderleistung einschaltet, die einem Pumpendruck unterhalb des ersten Druckniveaus entspricht.

7. Wanne nach einem der Ansprüche 1 bis 6, dadurch gekennzeichnet, daß jeweils das Absperrventil (14, 16) in der Überströmleitung (13) und deren Zweigleitung (15) jeweils mit einem Stellantrieb versehen ist, der über den Niveaufühler (23) ansteuerbar ausgebildet ist und zwar so, daß beim Überschreiten der Mindestfüllhöhe das Absperrventil (14) in der Überströmleitung (13') öffnet und das Absperrventil (16) in der Zweigleitung (15) wenigstens teilweise schließt.

8. Wanne nach einem der Ansprüche 1 bis 7, dadurch gekennzeichnet, daß die mit dem Wanneninnenraum in Verbindung stehende Ansaugöffnung der Saugleitung (9) durch die wannenseitige Öffnung (2) einer kammerartigen Wannenablaufarmatur (10) gebildet wird, in der der Wannenverschluß (11) unterhalb der Einmündung der Saugleitung (9) angeordnet ist, daß die Überströmleitung (13) oberhalb des Wannenverschlusses (11) und die Zweigleitung (15) unterhalb des Wannenverschlusses (11) in die Wannenverschlußarmatur (10) einmünden.

9. Wanne nach einem der Ansprüche 1 bis 8, dadurch gekennzeichnet, daß die Wannenablaufarmatur (10) eine unterhalb des Wannenbodens angeordnete Strömungskammer (50) aufweist, die mit einer oberen Öffnung versehen ist, die als Ablauföffnung (2) in den Wanneninnenraum mündet und die mit einer unteren Öffnung (52) versehen ist, die in das Ablaufrohr (3) mündet und die mit einem von außen betätigbaten Ventilkörper (53) verschließbar ist, daß die Strömungskammer (50) oberhalb des Ventilkörpers (53) zwei vorzugsweise diametral gegenüberliegend angeordnete Strömungsöffnungen (54, 55) aufweist, von denen eine an eine Zulaufleitung und die andere an eine Saugleitung anschließbar sind.

10. Wanne nach Anspruch 1 bis 9, dadurch gekennzeichnet, daß die an die Zulaufleitung anschließbare Öffnung (55) einen in die Strömungskammer (50) gegen die andere Öffnung (54) hineinragenden Rohransatz aufweist.

11. Wanne nach Anspruch 9 oder 10, dadurch gekennzeichnet, daß die mit dem Wanneninnenraum verbundene Ausaugöffnung (2) über einen Rohreinsatz (56) mit der darunterliegenden Ablauföffnung (22) verbunden ist, der einen geringeren Durchmesser als der Innendurchmesser der Strömungskammer (50) aufweist und daß die Wandung des Rohreinsatzes (56) auf Seiten der mit der Saugleitung verbindbaren Öffnung (54) der Strömungskammer (50) mit wenigstens einer Durchtrittsöffnung (57) versehen ist.

## Claims

1. A bath tub with at least one nozzle (6) built into the wall region for introducing a water-air mixture which nozzle is connected to an air-supply-pipe (12) and which nozzle is connected via a pressure line (7) to the pressure side of a pump (8), the suction side of which communicates with a suction opening in the base region of the tub via a pipe (9), and viewed in the direction of flow with a feed means (18) for a cleaning agent is provided before the nozzle (6), characterised in that the part of the nozzle (6) which communicates with the pump (8) via the pressure line (7) and which is seperate from the air-supply-pipe (12) is designed to be able to be closed off from the interior of the bath and is connected to an overflow pipe (13) which is provided with a closable venting opening (22) and which communicates with the suction line (9) of the pump (8) so that it can be blocked off vis a tub waste-pipe fitting which contains the suction opening (2).

2. A tub according to claim 1, characterised in that the overflow pipe (13) can be closed off from the suction line (9) by a preferably controllable shut-off valve (14).

3. A tub according to Claim 1 or 2, characterised in that the overflow pipe is provided with a branch pipe (15) in the direction of flow behind the nozzle (6), which pipe can be closed by a preferably controllable shut-off valve (16) and opens into the tub drain pipe (3).

4. A tub according to one of Claims 1 to 3, characterised in that the vent opening (20) is designed to be able to be blocked off by a shut-off valve (22), preferably a float valve.

5. A tub according to one of Claims 1 to 4, characterised in that the nozzle (6) has a blocking member which keeps the pressure line (7) closed from the interior of the tub, at a pump pressure of between zero and a first low pressure level and opens automatically if the first pressure level is exceeded.

6. A tub according to one of Claims 1 to 5, characterised in that a pump (8) which has a speed which can be altered, preferably a continuously adjustable pump, is provided, that a level sensor (23) is provided which when a minimum filling height is exceeded in the bath (1) switches on the drive motor of the pump (8) to a pumping capacity which, corresponds to a pump pressure below the first pressure level.

7. A tub according to one of Claims 1 to 6, characterised in that in each case the shut-off valve (14, 16) in the overflow pipe (13) and, the branch pipe (15) thereof is provided in each case with an actuating drive which is designed to be able to be controlled by means of the level sensor (23), such that when the minimum filling height is exceeded the shut-off valve (14) in the overflow pipe (13') opens and the shut-off valve (16) in the branch pipe (15) at least partially closes.

8. A tub according to one of Claims 1 to 7, characterised in that the suction opening of the suction line (9) which communicates with the interior of the bath is formed by the tub-side opening (2) of a chamber-like tub waste-pipe fitting (10) in which the bath closure (11) is located beneath the opening of, the suction line (9), that the overflow pipe (13) opens into the bath closure fitting (10) above the bath closure (11) and the branch pipe (15) below the bath closure (11).

9. A tub according to one of Claims 1 to 8, characterised in that the bath waste-pipe fitting (10) has a flow chamber (50) located beneath the base of the bath, which chamber is provided with an upper opening which opens into the interior of the tub as a drainage opening (2) and which is provided with a lower opening (52) which opens into the waste-pipe (3) and which can be closed by means of a valve member (53) which is externally actuated, that the flow chamber,(50) has, two preferably diametrically opposed flow openings (54, 55) above the valve member (53), one of which openings can be connected to an inlet pipe and the other to a suction pipe.

10. A tub according to Claims 1 to 9, characterised in that the opening (55) which can be connected to the inlet pipe has a pipe attachment which projects into the flow chamber (50) towards the other opening (54).

11. A tub according to Claim 9 or 10, characterised in that the suction opening (2) connected with the interior of the tub is connected to the underlying waste-pipe opening (22) via a pipe insert (56) which has a smaller diameter than the internal diameter of the flow chamber (50) and that the wall of the pipe insert (56) on sides of the opening (54) of the flow chamber (50) which can be connected to the suction line is provided with at least one passage opening (57).

## Revendications

1. Baignore comportant au moins une buse (6) d'introduction d'un mélange eau-air qui est incorporée dans la paroi et qui est reliée avec une conduite tubulaire (12) d'alimentation d'air et par l'intermédiaire d'une conduite de refoulement (7), avec le côte refoulement d'une pompe (8) dont le côté aspiration est relié par l'intermédiaire d'une conduite tubulaire (9), avec une ouverture d'aspiration située dans la zone du fond de le baignoire et avant la buse (6), vu dans la direction du flux, avec une alimentation (18) en produit de nettoyage. baignoire caractérisée, par le fait que la partie de la buse (6) qui est reliée. par l'intermédiaire de la conduite de refoulement (7) avec la pompe (8), est séparée de la conduite tubulaire (12) d'alimentation d'air et est concue obturable en direction de l'espace intérieur de la baignoire et est reliée à une conduite de débordement (13) qui est munie d'une ouverture (22) de purge d'air, obturable, et qui est en liaison verrouillable, par l'intermédiaire d'une bonde de vidange de la baignoire contenant l'ouverture de vidange (2), avec la conduite d'aspiration (9) da la pompe (8).

2. Baignoire selon la revendication 1, caractérisée par le fait que la conduite de débordement (13) peut être obturée à l'égard de la conduite d'aspiration (9) par une vanne d'arrêt (14), pouvant de préférence être commandée.

3. Baignoire selon la revendication 1 ou 2, caractérisée par le fait que la conduite de débordement présente, derrière la buse (6) dans la direction du flux, une conduite de dérivation (15) qui est obturable par une vanne d'arrêt (16), pouvant de préférence être commandée, et qui débouche dans le tuyau (3) de vidange de la baignoire.

4. Baignoire selon l'une des revendications 1 à 3, caractérisée par le fait que l'ouverture (20) de purge de l'air est conçue verrouillable au moyen d'un robinet d'arrêt (22), de préférence un robinet à flotteur.

5. Baignoire selon l'une des revendications 1 à 4, caractérisée par le fait que la buse (6) présente un organe de verrouillage qui maintient fermée la conduite de refoulement (7) par rapport à l'espace intérieur de la baignoire pour une pression de la pompe comprise entre zéro et une première valeur faible de la pression et qui s'ouvre automatiquement lors du dépassement de cette première valeur de la pression.

6. Baignoire selon l'une des revendications 1 à 5, caractérisée par le fait qu'il est prévu une pompe (8) de vitesse de rotation modifiable, réglable de préférence en continu, et par le fait qu'il est prévu un capteur de niveau (23) qui, lors du dépassement, dans la baignoire (1), d'une hauteur de remplissage minimale, met en circuit le moteur d'entraînement de la pompe (8) sur un débit qui correspond à une pression de la pompe inférieure à la première valeur de pression.

7. Baignoire selon l'une des revendications 1 à 6, caractérisée par le fait que chacune des vannes d'arrêt (14, 16) prévues sur la conduite de débordement (13) et sur sa conduite de dérivation (15) est munie d'un mécanisme d'entraînement en position conçu pour être commandé par le capteur de niveau (23) et, de façon plus précise, de façon que, lors du dépassement de la hauteur de remplissage minimale, la vanne d'arrêt (14) prévue sur la conduite de débordement (13') s'ouvre et que la vanne d'arrêt (16) prévue sur la conduite de dérivation (15) se ferme au moins partiellement.

8. Baignoire selon l'une des revendications 1 à 7, caractérisée par le fait que l'ouverture d'aspiration, reliée à l'espace intérieur de la baignoire, de la conduite d'aspiration (9) est formée par l'ouverture (2), côté baignoire, d'une bonde (10), en forme de chambre, de vidange de la baignoire, bonde dans laquelle l'obturateur (11) de la baignoire est disposé en-dessous de l'endroit où débouche la conduite d'aspiration (9), et que la conduite de débordement (13) débouche au-dessus de l'obturateur (11) situé dans la bonde (10) de vidange de la baignoire et la conduite de dérivation (15), au-dessous de cet obturateur.

9. Baignoire selon l'une des revendications 1 à 8, caractérisée par le fait que la bonde (10) de vidange de la baignoire présente une chambre d'écoulement (50), disposée en-dessous du fond de la baignoire, qui est munie d'une ouverture supérieure débouchant, dans l'espace intérieur de la baignoire, en tant qu'ouverture de vidange (2) et d'une ouverture inférieure (52) qui débouche dans le tuyau de vidange (3) et peut être obturée par un obturateur (53) manoeuvrable de l'extérieur, et que la chambre d'écoulement (50) présente, audessus de l'obturateur (53), deux ouvertures d'écoulement (54, 55) qui de préférence sont situées diamétralement en face l'une de l'autre et dont l'une peut être reliée à une conduite d'arrivée et l'autre, à une conduite d'aspiration.

10. Baignoire selon l'une des revendications 1 à 9, caractérisée par le fait que l'ouverture (55) qui peut être reliée la conduite d'arrivée présente un embout tubulaire qui pénètre dans la chambre d'écoulement (50) en direction de l'autre ouverture (54).

11. Baignoire selon la revendication 9 ou 10, caractérisée par le fait que l'ouverture d'aspiration (2), reliée à l'espace intérieur de la baignoire, est reliée avec l'ouverture de vidange (22) située par dessous par l'intermédiaire d'une garniture tubulaire (56) qui présente un diamètre inférieur au diamètre intérieur de la chambre d'écoulement (50) et que la paroi de la garniture tubulaire (56) présente, du côté de l'ouverture (54) de la chambre d'écoulement (50) qui peut être reliée à la conduite d'aspiration, au moins une ouverture de passage (57).
